Europäisches Patentamt

European Patent Office

Office européen des brevets

(11) Veröffentlichungsnummer: **0 291 494 B1**

(12) **EUROPÄISCHE PATENTSCHRIFT**

(45) Veröffentlichungstag der Patentschrift: **11.11.92**

(51) Int. Cl.⁵: **D21C 11/00,** C07D 307/50, D21C 3/22

(21) Anmeldenummer: **88890108.9**

(22) Anmeldetag: **05.05.88**

(54) Kombiniertes Verfahren zur thermischen und chemischen Behandlung von lignocellulosehaltiger Biomasse und zur Gewinnung von Furfural.

(30) Priorität: **12.05.87 AT 1188/87**

(43) Veröffentlichungstag der Anmeldung:
**17.11.88 Patentblatt 88/46**

(45) Bekanntmachung des Hinweises auf die Patenterteilung:
**11.11.92 Patentblatt 92/46**

(84) Benannte Vertragsstaaten:
**CH DE ES FR IT LI SE**

(56) Entgegenhaltungen:
**EP-A- 0 038 317**
**EP-A- 0 124 507**
**DE-A- 2 610 682**

(73) Patentinhaber: **VOEST-ALPINE INDUSTRIEAN-LAGENBAU GmbH**
**Turmstrasse 44**
**A-4020 Linz(AT)**

(72) Erfinder: **Avignon, Gerard, Ing.Mag.**
**6, Rue J.F. Blade**
**F-47000 Agen(FR)**
Erfinder: **Jaeggle, Wolfgang, Dipl.-Ing.**
**Mörikestrasse 48**
**W-7981 Bodnegg(DE)**
Erfinder: **Steinmüller, Horst, Dipl.-Ing.**
**Weberstrasse 16**
**A-4060 Leonding(AT)**
Erfinder: **Steiner, Thomas, Dipl.-Ing.**
**Harterfeldstrasse 4**
**A-4060 Leonding(AT)**

(74) Vertreter: **Wolfram, Gustav**
**Patentanwälte Sonn, Pawloy, Weinzinger &**
**Wolfram Riemergasse 14**
**A-1010 Wien(AT)**

## Beschreibung

Die Erfindung betrifft ein kombiniertes Verfahren zur thermischen und chemischen Behandlung von lignocellulosehaltiger Biomasse und zur Gewinnung von Furfural und cellulosehaltigen Fasermassen, sowie eine Anlage zur Durchführung des Verfahrens.

Ein solches kombiniertes Verfahren ist beispielsweise aus der DE-A - 34 35 451 und aus der EP-B - 0 038 317 bekannt. In der DE-A - 34 35 451 ist zur Herstellung von Zellstoff und Furfural ein zweistufiges Verfahren mit Vorhydrolyse und anschließendem chemischen Aufschluß beschrieben. Dabei wird das aufzuschließende Material in Gegenwart einer verdünnten Säure bei Temperaturen unter 150°C so lange erhitzt, bis die enthaltenen Pentosane zu Pentosen hydrolysiert sind. Danach wird ein Aufschlußmittel zugeführt und die Temperatur schnell auf über 160°C gesteigert und dort so lange gehalten, bis der Aufschluß beendet ist. Dabei wird das sich aus den Pentosen bildende Furfural abgeführt.

In der EP-B - 0 038 317 ist ein Verfahren zur Gewinnung von Furfural und anderen organischen Verbindungen aus sauren Hydrolysaten von Pflanzen, insbesondere Sulfitablaugen, beschrieben. Dabei werden die in den Ablaugen enthaltenen Pentosen durch Erhitzen in einem Reaktor auf Temperaturen bis zu 300°C zu Furfural dehydratisiert.

Der Reaktionsablauf der Bildung von Furfural aus in lignocellulosehaltiger Biomasse enthaltenen Pentosanen umfaßt zwei durch Zugabe von Säuren begünstigte Reaktionsschritte. Der erste besteht in einer Wasseraufnahme, wobei die Pentosane durch Hydrolyse in Pentosen übergeführt werden, und der zweite in einer Dehydratisierung, wodurch die Pentosen in Furfural umgewandelt werden. Beim Verfahren nach der DE-A -34 35 451 laufen diese beiden Reaktionsschritte während des chemischen Aufschlusses der Biomasse ab. Durch die hierbei drastischen Reaktionsbedingungen kommt es aber teilweise zur Bildung von Kondensationsprodukten aus Furfural und zu Zersetzungsreaktionen der Pentosane. Die gleichen Nachteile weist auch das Verfahren nach der EP-B - 0 038 317 auf, bei dem Furfural erst nach dem Aufschluß aus der Ablauge gebildet wird. Bei beiden Verfahren ergibt sich daher gegenüber der theoretisch maximal möglichen Furfuralausbeute eine nur stark verminderte Ausbeute durch Bildung von Kondensationsprodukten, die sich im Falle der Herstellung von gebleichtem Sulfitzellstoff auch auf diesen nachteilig auswirken.

Die Erfindung bezweckt die Vermeidung dieser Schwierigkeiten und Nachteile und stellt sich die Aufgabe, ein kombiniertes Verfahren der eingangs beschriebenen Art sowie eine Anlage zur Durchführung des Verfahrens zu schaffen, durch die die Bildung von Folgeprodukten aus Pentosen im Kochprozeß selbst verhindert werden soll, so daß die Ausbeute an Furfural erhöht wird, und durch die bei der Herstellung von gebleichtem Sulfitzellstoff nur ein geringer Bleichmitteleinsatz erforderlich ist.

Diese Aufgabe wird erfindungsgemäß dadurch gelöst, daß

- in einen Kochflüssigkeit enthaltenden Kocher lignocellulosehaltige Biomasse kontinuierlich zugeführt und nach Erhitzen der Biomasse in einer Aufheizzone sowie Führen der erhitzten Biomasse durch eine anschließende Haltezone dem Kocher Zellstoff oder vorbehandelte Biomasse kontinuierlich entnommen wird,
- Kochflüssigkeit dem Kocher kontinuierlich entnommen wird,
- die Kochflüssigkeit einer Furfuralerzeugungsanlage kontinuierlich zugeführt wird und
- die von Pentosen bzw. Furfural zumindest größtenteils befreite Kochflüssigkeit in den Kocher kontinuierlich wieder rückgeführt wird, wobei die Erhitzung durch Direkt-Dampf oder über die Kochflüssigkeit erfolgen kann.

Durch die kontinuierliche Entfernung der Pentosen aus dem Kocher wird die Weiterreaktion zu Furfural und dessen Kondensationsprodukten im Kocher selbst verhindert. Dadurch wird einerseits die Ausbeute an Pentosen und damit an Furfural stark erhöht und andererseits bei der Herstellung von Zellstoff dessen Reinheit verbessert, wodurch mit einem geringeren Bleichmitteleinsatz in der weiteren Verarbeitung das Auslangen gefunden wird. Die Rückführung der Kochflüssigkeit ermöglicht eine kontinuierliche Abtrennung der Pentosen, ohne dabei die thermische Behandlung der lignocellulosehaltigen Biomasse unterbrechen zu müssen. Außerdem wird ein Verarmen des Kochers an Kochflüssigkeit vermieden.

Gemäß einer bevorzugten Ausführungsform wird Kochflüssigkeit entnommen, deren Konzentration an Pentosen größer als 5 g/l ist und bei der die Zersetzung der Pentosen in der Kochflüssigkeit unter 10 % liegt. Durch die Wahl der Pentosen-Konzentration ergibt sich eine bestimmte Entnahmestelle am Kocher, wodurch die Abtrennung der Pentosen den jeweiligen Reaktionsbedingungen im Kocher angepaßt und die Ausbeute an Pentosen bzw. an Furfural optimiert werden kann.

Vorteilhaft wird bei der Herstellung von Zellstoff nach dem Sulfitverfahren die Kochflüssigkeit aus dem Kocher mit einer Temperatur im Bereich von 100 bis 150°C, vorzugsweise 110 bis 130°C, abgezogen und der Furfuralerzeugungsanlage zugeführt. Durch die drastischen Reaktionsbedingungen bei der Herstellung von Zellstoff nach dem Sulfitverfahren würde eine Entnahme von Kochflüssigkeit aus einer Zone höherer Temperatur zu unkontrollierten Weiterreaktionen der Pentosen und damit zu einer verminderten Ausbeute führen.

Desgleichen ist es günstig, wenn zur Herstellung durch Vorhydrolyse vorbehandelter Biomasse bei Anwendung einer Wasservorhydrolyse die Kochflüssigkeit mit einer Temperatur im Bereich von 120 bis 200°C, vorzugsweise 130 bis 170°C, entnommen wird, bzw. bei Anwendung einer Säurevorhydrolyse die Kochflüssigkeit mit einer Temperatur zwischen 100 und 150°C, vorzugsweise 110 bis 130°C entnommen wird.

Nach einer weiteren bevorzugten Ausführungsform werden in der Furfuralerzeugungsanlage die in der Kochflüssigkeit enthaltenen Pentosen zu Furfural dehydratisiert, das erhaltene Substrat einer Wasserdampfdestillation unterworfen, die zurückbleibende Kochflüssigkeit in den Kocher rückgeführt und die kondensierten Brüden einer einoder mehrstufigen Destillation zur Abtrennung des Furfurals unterworfen.

Vorteilhaft ist auch, wenn die bei der ein- oder mehrstufigen Destillation anfallende Rückstandsflüssigkeit in den Kocher rückgeführt wird.

Weiters ist vorteilhaft, wenn bei der Herstellung von Zellstoff in der Furfuralerzeugungsanlage anfallendes $SO_2$ in den Kocher rückgeführt wird. Dadurch wird ein Verarmen der im Kocher befindlichen Kochflüssigkeit an Schwefeldioxid vermieden, und es erübrigt sich ein ständiges Ergänzen jener $SO_2$-Menge, die mit der Kochflüssigkeit in die Furfuralerzeugungsanlage gelangt.

Bevorzugt wird die Pentosen-enthaltende Kochflüssigkeit durch die Furfuralerzeugungsanlage mit einer Gesamtverweilzeit in einem Bereich zwischen 10 und 60 min, vorzugsweise zwischen 10 und 25 min, geführt, wobei die Kochflüssigkeit auf eine Temperatur zwischen 130 und 180°C, vorzugsweise zwischen 150 und 180°C, aufgeheizt und in diesem Bereich gehalten wird. Naturgemäß schwankt der Gehalt der Kochflüssigkeit an Pentosen während der Aufheizphase. Durch Festlegen einer Gesamtverweilzeit in der Furfuralerzeugungsanlage bei einer bestimmten Temperatur kann die Reaktionsbedingung der Dehydratisierung dem jeweiligen Gehalt an Pentosen angepaßt und damit die Furfuralausbeute verbessert werden.

Eine Anlage zur Durchführung des Verfahrens umfaßt einen Kocher, in den eine Zuführungsleitung für lignocellulosehaltige Biomasse und eine Leitung für Dampf einmündet und an den eine Ableitung für im Kocher thermisch behandelte Biomasse sowie eine zu einem Wärmetauscher führende Zweigleitung angeschlossen ist, von dem eine in den Kocher mündende Rückleitung für die Kochflüssigkeit ausgeht, und eine, einen Rohrreaktor, eine Reaktionskolonne und eine nachfolgende Destillationseinrichtung aufweisende Furfuralerzeugungsanlage, deren Rohrreaktor mit einer Zulaufleitung und deren Reaktionskolonne mit einer Rücklaufleitung mit dem Kocher leitungsmäßig verbindbar sind.

Gemäß einer zweckmäßigen Ausführungsform geht die Zweigleitung von einer Stelle des Kochers aus, an der die Konzentration an Pentosen größer als 5 g/l ist und bei der die Zersetzung der Pentosen in der Kochflüssigkeit unter 10 % liegt.

Nach einer weiteren zweckmäßigen Ausführungsform ist die Destillationseinrichtung zur Rückführung von vom Furfural abgetrennter Flüssigkeit mit dem Kocher leitungsmäßig verbindbar. Damit kann der von Furfural befreite Destillationsrückstand der Destillationseinrichtung wieder in den Kocher zurückgeleitet werden.

Weiters ist vorteilhaft, wenn eine Brüden-Ableitung der Reaktionskolonne in einen Sammelbehälter mündet, der mit der Destillationseinrichtung verbunden ist, wobei der Sammelbehälter einen Gasraum aufweist, der leitungsmäßig mit dem Kocher verbindbar ist. Dadurch kann das bei der Herstellung von Sulfitzellstoff anfallende $SO_2$ in den Kocher zurückgeleitet werden.

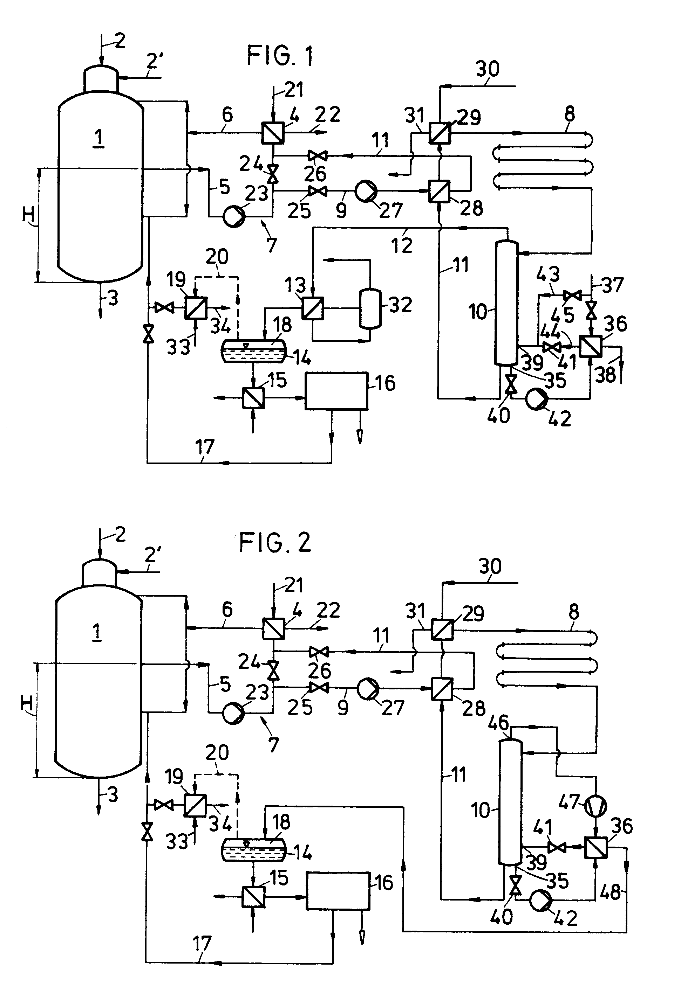

Das erfindungsgemäße Verfahren und eine Anlage zur Durchführung des Verfahrens sind nachfolgend anhand der Fig. 1 und 2 näher erläutert. Beide Figuren zeigen ein Blockschema der Anlage nach je einer Ausführungsform.

Gemäß Fig. 1 mündet in einen Kocher 1 eine Zuführungsleitung 2 zur Beschickung des Kochers mit Kochflüssigkeit und Biomasse. Am oberen Ende des Kochers 1 mündet eine Dampfleitung 2′ zur Direktaufheizung der zugeführten Biomasse. Weiters sind am Kocher 1 eine Ableitung 3 zur Entnahme von vorbehandelter Biomasse bzw. Sulfitzellstoff sowie in einer bestimmten Höhe H des Kochers, wie später noch erläutert wird, eine zu einem für die Aufheizung der Kochflüssigkeit vorgesehenen Wärmetauscher 4 führende Zweigleitung 5 angeschlossen. Vom Wärmetauscher 4 geht eine Rückleitung 6 für die Kochflüssigkeit aus, die in den Kocher 1 mündet und somit einen Heizkreislauf 7 schließt. An die Zweigleitung 5 sind bypassartig ein Rohrreaktor 8 über eine Zulaufleitung 9 und eine zum Rohrreaktor 8 in Serie geschaltete Reaktionskolonne 10 über eine Rücklaufleitung 11 angeschlossen. Von der Reaktionskolonne führt eine Brüden-Ableitung 12 über einen Wärmetauscher 13, einen Sammelbehälter 14 und über einen weiteren Wärmetauscher 15 zu einer Destillationseinrichtung 16, die über eine Leitung 17 für den Destillationsrückstand mit dem Kocher 1 verbunden ist. Wird die oben beschriebene Anlage zur Herstellung von Sulfitzellstoff eingesetzt, ist zweckmäßig zusätzlich der Gasraum 18 des Sammelbehälters 14 über einen Wärmetauscher 19 mit dem Kocher 1 über eine in den Figuren mit strichlierten Linien dargestellte Leitung 20 verbindbar.

Das erfindungsgemäße Verfahren wird wie folgt durchgeführt:

Dem Kocher 1 wird über die Zuführungsleitung 2 lignocellulosehaltige Biomasse und Kochflüssigkeit zugeführt und der Inhalt des Kochers mit Direktdampf aufgeheizt. Über die Zweigleitung 5 wird zur Temperaturfeineinstellung Kochlauge aus dem Kocher 1 abgezogen und in den mit Hochdruckdampf (veranschaulicht durch die Pfeile 21, 22) versorgten Wärmetauscher 4 geleitet, dort erhitzt und über die Rückleitung 6 wieder in den Kocher 1 zurückgeleitet. Die Umwälzung der Kochlauge wird von einer in der Zweigleitung 5 vorgesehenen Pumpe 23 besorgt. Zwischen der Abzweigung der Zulaufleitung 9 und der Einmündung der Rücklaufleitung 11 ist in der Zweigleitung ein Ventil 24 vorgesehen, das bei der direkten Umwälzung der Kochflüssigkeit über den Wärmetauscher 4 in der Anfahrphase des Kochers 1 geöffnet ist, wogegen jeweils ein in der Zulaufleitung 9 und in der Rücklaufleitung 11 befindliches Ventil 25, 26 geschlossen ist.

Sobald im Kocher Pentosane in einer vorbestimmten Konzentration zu Pentosen hydrolysiert und von der Kochflüssigkeit gelöst sind, wird das in der Zweigleitung eingebaute Ventil 24 geschlossen und werden die in der Zu-und Rücklaufleitung vorgesehenen Ventile 25 und 26 geöffnet, so daß Kochflüssigkeit kontinuierlich über die Zulaufleitung 9 in den Rohrreaktor 8 mit Hilfe einer Pumpe 27, die in der Zulaufleitung 9 angeordnet ist, gepumpt wird. Während der Umwälzung der Kochflüssigkeit in der Furfuralerzeugungsanlage 8, 10, 16 wird dem Kocher 1 kontinuierlich lignocellulosehaltige Biomasse zugeführt und die fertig behandelte Biomasse (bzw. Zellstoff) kontinuierlich entnommen.

Die Höhe H der Entnahmestelle der Zweigleitung 5 am Kocher 1 ist so gewählt, daß die Konzentration an Pentosen in der Kochflüssigkeit bereits größer als 5 g/l ist und die Zersetzung der Pentosen in der Kochflüssigkeit unter 10 % liegt. Bei der Herstellung von vorbehandelter Biomasse wird bei der Temperaturführung innerhalb des Kochers 1 darauf geachtet, daß sich die Temperatur der entnommenen Kochflüssigkeit bei Anwendung einer Wasservorhydrolyse im Bereich von 120 bis 200°C, vorzugsweise 130 bis 170°C, und bei Anwendung einer Säurevorhydrolyse im Bereich von 100 bis 150°C, vorzugsweise 110 bis 130°C, bewegt.

Die über die Zulaufleitung 9 abgezweigte Kochflüssigkeit wird in zwei ebenfalls in der Zulaufleitung vorgesehenen Wärmetauschern 28 und 29, von denen einer (28) mit über die Rücklaufleitung 11 rücklaufender Kochflüssigkeit und der andere (29) mit Hochdruckdampf (veranschaulicht durch die Pfeile 30 und 31) versorgt werden, geleitet und dabei auf die für die Dehydratisierung der Pentosen zu Furfural notwendige Temperatur gebracht. Dieser Reaktionsschritt erfolgt größtenteils im Rohrreaktor 8 und teilweise auch noch in der Reaktionskolonne 10, in die die Kochflüssigkeit anschließend geleitet wird. In der Reaktionskolonne 10 wird mit Wasserdampf das gebildete Furfural aus der Kochflüssigkeit ausgetrieben, d.h. abgestrippt, über die Brüden-Ableitung 12 in den Wärmetauscher 13 geleitet, dort unter Bildung von Sekundärdampf kondensiert und die dabei gebildete wässerige Furfurallösung im Sammelbehälter 14 aufgefangen.

Der sich im Wärmetauscher 13 bildende Sekundärdampf wird in einem Kühler 32 kondensiert und dem Wärmetauscher 13 wieder zugeführt. Die von Furfural befreite und am unteren Ende der Reaktionskolonne 10 abgezogene Kochflüssigkeit wird mittels der Rückleitung 11 kontinuierlich über die Wärmetauscher 28 und 4 in den Kocher rückgeführt.

Wird die Anlage zur Herstellung von Sulfitzellstoff betrieben, wird bei der Temperaturführung innerhalb des Kochers 1 darauf geachtet, daß sich die Temperatur der entnommenen Kochflüssigkeit im Bereich von 100 bis 150°C, vorzugsweise 110 bis 130°C bewegt. Im Gasraum 18 des Sammelbehälters 14 anfallendes Schwefeldioxid wird im Wärmetauscher 19, der mit Hochdruckdampf, veranschaulicht durch die Pfeile 33 und 34, versorgt wird, erhitzt und anschließend wieder in den Kocher 1 rückgeleitet.

Die wässerige Furfurallösung im Sammelbehälter 14 wird im Wärmetauscher 15 unter Bildung von Sekundärdampf gekühlt und der Furfuraldestillationseinrichtung 16 zugeführt, in der in einer oder mehreren Stufen Furfural gewonnen wird. Der dabei anfallende Destillationsrückstand wird über die Leitung 17 wieder in den Kocher 1 zurückgeleitet.

Die Wärmeversorgung der Reaktionskolonne 10 kann sowohl bei der Herstellung von Zellstoff als auch zur Herstellung von vorbehandelter Biomasse zweckmäßigerweise in zwei Varianten erfolgen:
Gemäß Fig. 1 wird die Kochflüssigkeit der Reaktionskolonne am unteren Ende 35 derselben entnommen, im Wärmetauscher 36 mit Hochdruckdampf (veranschaulicht durch die Pfeile 37, 38) erhitzt und wieder in die Reaktionskolonne 10 nahe deren unterem Ende 39 zurückgeleitet. Die Umwälzung wird mittels Ventile 40 und 41 und einer Pumpe 42 gesteuert. Von der Hochdruckdampfzuleitung 37 führt eine Hochdruckdampf-Zweigleitung 43 in die vom Wärmetauscher 36 ausgehende Rückführung 44, wobei in der Hochdruckdampf-Zweigleitung 43 ein Regelventil 45 vorgesehen ist. Durch ein Zusammenspiel der Ventile 40, 41 und 45 kann Heißdampf sowohl zur Wärmeversorgung des Wärmetauschers 36 als auch zur Einspeisung in die Reaktionskolonne 10 herangezogen werden.

Gemäß Fig. 2 werden die die Reaktionskolonne 10 am oberen Ende 46 verlassenden Brüden in einem Kompressor 47 verdichtet und zur Wärmeversorgung des Wärmetauschers 36 verwendet. Die im Wärmetauscher 36 kondensierten Brüden werden danach über eine Leitung 48 in den Sammelbehälter 14 geleitet und, wie oben beschrieben, aufgearbeitet.

**Patentansprüche**

1. Kombiniertes Verfahren zur thermischen und chemischen Behandlung von lignocellulosehaltiger Biomasse und zur Gewinnung von Furfural und cellulosehaltigen Fasermassen, dadurch gekennzeichnet, daß
   - in einen Kochflüssigkeit enthaltenden Kocher (1) lignocellulosehaltige Biomasse kontinuierlich zugeführt und nach Erhitzen der Biomasse in einer Aufheizzone sowie Führen der erhitzten Biomasse durch eine anschließende Haltezone dem Kocher (1) Zellstoff oder vorbehandelte Biomasse kontinuierlich entnommen wird,
   - Kochflüssigkeit dem Kocher (1) kontinuierlich entnommen wird,
   - die Kochflüssigkeit einer Furfuralerzeugungsanlage (8, 10, 16) kontinuierlich zugeführt wird und
   - die von Pentosen bzw. Furfural zumindest größtenteils befreite Kochflüssigkeit in den Kocher (1) kontinuierlich wieder rückgeführt wird, wobei die Erhitzung durch Direkt-Dampf oder über die Kochflüssigkeit erfolgen kann.

2. Verfahren nach Anspruch 1, dadurch gekennzeichnet, daß Kochflüssigkeit entnommen wird, deren Konzentration an Pentosen größer als 5 g/l ist und bei der die Zersetzung der Pentosen in der Kochflüssigkeit unter 10 % liegt.

3. Verfahren nach Anspruch 2, dadurch gekennzeichnet, daß bei der Herstellung von Zellstoff nach dem Sulfitverfahren die Kochflüssigkeit aus dem Kocher (1) mit einer Temperatur im Bereich von 100 bis 150°C, vorzugsweise 110 bis 130°C, abgezogen und der Furfuralerzeugungsanlage (8, 10, 16) zugeführt wird.

4. Verfahren nach Anspruch 2, dadurch gekennzeichnet, daß zur Herstellung durch Vorhydrolyse vorbehandelter Biomasse bei Anwendung einer Wasservorhydrolyse die Kochflüssigkeit mit einer Temperatur im Bereich von 120 bis 200°C, vorzugsweise 130 bis 170°C, entnommen wird, bzw. bei Anwendung einer Säurevorhydrolyse die Kochflüssigkeit mit einer Temperatur zwischen 100 und 150°C, vorzugsweise 110 bis 130°C entnommen wird.

5. Verfahren nach Anspruch 1, dadurch gekennzeichnet, daß in der Furfuralerzeugungsanlage (8, 10, 16) die in der Kochflüssigkeit enthaltenen Pentosen zu Furfural dehydratisiert, das erhaltene Substrat einer Wasserdampfdestillation unterworfen, die zurückbleibende Kochflüssigkeit in den Kocher (1) rückgeführt und die kondensierten Brüden einer ein-oder mehrstufigen Destillation zur Abtrennung des Furfurals unterworfen werden.

6. Verfahren nach Anspruch 5, dadurch gekennzeichnet, daß die bei der ein- oder mehrstufigen Destillation anfallende Rückstandsflüssigkeit in den Kocher (1) rückgeführt wird.

7. Verfahren nach Anspruch 3, dadurch gekennzeichnet, daß bei der Herstellung von Zellstoff in der Furfuralerzeugungsanlage (8, 10, 16) anfallendes $SO_2$ in den Kocher (1) rückgeführt wird.

8. Verfahren nach einem oder mehreren der Ansprüche 1 bis 7, dadurch gekennzeichnet, daß die Pentosen-enthaltende Kochflüssigkeit durch die Furfuralerzeugungsanlage (8, 10, 16) mit einer Gesamtverweilzeit in einem Bereich zwischen 10 und 60 min, vorzugsweise zwischen 10 und 25 min, geführt wird, wobei die Kochflüssigkeit auf eine Temperatur zwischen 130 und 180°C, vorzugsweise zwischen 150 und 180°C, aufgeheizt und in diesem Bereich gehalten wird.

9. Anlage zur Durchführung des Verfahrens nach einem oder mehreren der Ansprüche 1 bis 8, umfassend einen Kocher (1), in den eine Zuführungsleitung (2) für lignocellulosehaltige Biomasse und eine Leitung (2') für Dampf einmündet und an den eine Ableitung (3) für im Kocher (1) thermisch behandelte Biomasse sowie eine zu einem Wärmetauscher (4) führende Zweigleitung (5) angeschlossen ist, von dem eine in den Kocher (1) mündende Rückleitung (6) für die Kochflüssigkeit ausgeht, und eine, einen Rohrreaktor (8), eine Reaktionskolonne (10) und eine nachfolgende Destillationseinrichtung (16) aufweisende Furfuralerzeugungsanlage, deren Rohrreaktor (8) mit einer Zulaufleitung (9) und deren Reaktionskolonne (10) mit einer Rücklaufleitung (11) mit dem Kocher (1) leitungsmäßig verbindbar sind.

10. Anlage nach Anspruch 9, dadurch gekennzeichnet, daß die Zweigleitung (5) von einer Stelle (H) des Kochers (1) ausgeht, an der die Konzentration an Pentosen größer als 5 g/l ist und bei der die Zersetzung der Pentosen in der Kochflüssigkeit unter 10 % liegt.

11. Anlage nach Anspruch 9 oder 10, dadurch gekennzeichnet, daß die Destillationseinrichtung (16) zur Rückführung von vom Furfural abgetrennter Flüssigkeit mit dem Kocher (1) leitungsmäßig verbindbar ist.

12. Anlage nach einem oder mehreren der Ansprüche 9 bis 11, dadurch gekennzeichnet, daß eine Brüden-Ableitung (12) der Reaktionskolonne (10) in einen Sammelbehälter (14) mündet, der mit der Destillationseinrichtung (16) verbunden ist, wobei der Sammelbehälter (14) einen Gasraum (18) aufweist, der leitungsmäßig mit dem Kocher (1) verbindbar ist.

## Claims

1. Combined process for thermally and chemically treating lignocellulose-containing biomass and for recovering furfural and cellulose-containing fibre masses, characterised in that
   - lignocellulose-containing biomass is continuously fed into a cooker (1) containing a cooking liquor and, after heating the biomass in a heating zone and guiding the heated biomass through a consecutive retention zone, cellulose or pre-treated biomass is continuously withdrawn from the cooker (1),
   - cooking liquor is continuously withdrawn from the cooker (1),
   - the cooking liquor is continuously supplied to a furfural production plant (8, 10, 16), and
   - the cooking liquor at least substantially freed from pentoses and furfural, respectively, is continuously returned into the cooker (1), wherein the heating may be effected by direct steam or via the cooking liquor.

2. A process according to claim 1, characterised in that cooking liquor is withdrawn, whose concentration of pentoses is higher than 5 g/l and in which the decomposition of the pentoses in the cooking liquor is below 10%.

3. A process according to claim 2, characterised in that in the production of cellulose according to the sulfite method the cooking liquor is withdrawn from the cooker (1) at a temperature in the range of 100 to 150°C, preferably 110 to 130°C and supplied to the furfural production plant (8, 10, 16).

4. A process according to claim 2, characterised in that for the preparation of biomass pretreated by pre-hydrolysis, when using a water pre-hydrolysis, the cooking liquor is withdrawn at a temperature in the range of 120 to 200°C, preferably 130 to 170°C, or when using an acid pre-hydrolysis, the cooking liquor is withdrawn at a temperature between 100 and 150°C, preferably 110 to 130°C.

5. A process according to claim 1, characterised in that in the furfural production plant (8, 10, 16) the pentoses contained in the cooking liquor are dehydrated to furfural, the substrate obtained is subjected to a steam distillation, the remaining cooking liquor is returned into the cooker (1), and the condensed exhaust vapors are subjected to a single- or multi-stage distillation for separating the furfural.

6. A process according to claim 5, characterised in that the residual liquid incurred in the single- or multi-stage distillation is returned into the cooker (1).

7. A process according to claim 3, characterised in that $SO_2$ incurred in the furfural production plant (8, 10, 16) at the production of cellulose is returned into the cooker (1).

8. A process according to one or several of claims 1 to 7, characterised in that the pentoses-containing cooking liquor is guided through the furfural production plant (8, 10, 16) with a total residence time in a range of between 10 and 60 min, preferably between 10 and 25 min, the cooking liquor being heated to a temperature of between 130 and 180°C, preferably between 150 and 180°C, and maintained in this range.

9. A plant for carrying out the process according to one or several of claims 1 to 8, comprising a cooker (1) into which a feed duct (2) for lignocellulose-containing biomass and a duct (2') for vapor enter and to which a discharge duct (3) for biomass thermally treated in the cooker (1) as well as a branch duct (5) leading to a heat exchanger (4) is connected, from which there departs a cooking liquor-return duct (6) entering into the cooker (1), and a furfural production plant comprising a pipe reactor (8), a reaction column (10) and a consecutive distillation device (16), whose pipe reactor (8) is capable of being flow-connected with the cooker (1) by a supply duct (9) and whose reaction column (10) is capable of being flowconnected with the cooker (1) by a return duct (11).

10. A plant according to claim 9, characterised in that the branch duct (5) departs from a site (H) of the cooker (1) at which the concentration of pentoses is higher than 5 g/l and at which the decomposition of the pentoses in the cooking liquor is below 10%.

11. A plant according to claim 9 or 10, characterised in that the distillation device (16) for returning the liquid separated from furfural is capable of being flow-connected with the cooker (1).

12. A plant according to one or several of claims 9 to 11, characterised in that an exhaust vapor discharge duct (12) of the reaction column (10) enters into a collecting basin (14), which is connected with the distillation device (16), wherein the collecting basin (14) comprises a gas space (18) capable of being flow-connected with the cooker (1).

**Revendications**

1. Procédé combiné pour le traitement thermique et chimique d'une biomasse contenant de la lignocellulose et pour l'obtention de furfural et de masses fibreuses contenant de la cellulose, caractérisé en ce que :
    - la biomasse contenant de la lignocellulose est introduite continuellement dans un lessiveur (1) contenant un liquide de lessivage et, après chauffage de la biomasse dans une zone de chauffage et envoi de la biomasse chauffée dans une zone de maintien voisine, de la pâte de cellulose ou la biomasse prétraitée est retirée continuellement du lessiveur (1),
    - le liquide de lessivage est retiré continuellement du lessiveur (1),
    - le liquide de lessivage est envoyé continuellement dans une installation de production de furfural (8, 10, 16), et
    - le liquide de lessivage débarrassé au moins en majeure partie des pentoses ou du furfural est renvoyé continuellement dans le lessiveur (1), le chauffage pouvant se faire par vapeur directe ou par le liquide de lessivage.

2. Procédé selon la revendication 1, caractérisé en ce que l'on retire un liquide de lessivage dont la concentration en pentoses est supérieure à 5 g/l et dans lequel la décomposition des pentoses dans le liquide de lessivage est inférieure à 10%.

3. Procédé selon la revendication 2, caractérisé en ce que, pour la fabrication de pâte de cellulose selon le procédé au sulfite, le liquide de lessivage et retiré du lessiveur (1) à une température située dans la plage de 100 à 150°C, de préférence de 110 à 130°C, et est envoyé dans l'installation de production de furfural (8, 10, 16).

4. Procédé selon la revendication 2, caractérisé en ce que, pour la fabrication d'une biomasse prétraitée par préhydrolyse, on retire un liquide de lessivage à une température située dans la plage de 120 à 200°C, de préférence de 130 à 170°C, lorsque l'on utilise une préhydrolyse à l'eau, et on retire un liquide de lessivage à une température comprise entre 100 et 150°C, de préférence entre 110 et 130°C, lorsque l'on utilise une préhydrolyse acide.

5. Procédé selon la revendication 1, caractérisé en ce que, dans l'installation de production de furfural (8, 10, 16), les pentoses contenus dans le liquide de lessivage sont déshydratés en furfural, le substrat obtenu est soumis à une distillation à la vapeur d'eau, le liquide de lessivage restant est renvoyé dans le lessiveur (1) et les vapeurs condensées sont soumises à une distillation à une ou plusieurs étapes pour la séparation du furfural.

6. Procédé selon la revendication 5, caractérisé en ce que le liquide résiduaire formé au cours de la distillation à une ou plusieurs étapes est renvoyé dans le lessiveur (1).

7. Procédé selon la revendication 3, caractérisé en ce que le $SO_2$ formé dans l'installation de production de furfural (8, 10, 16) lors de la fabrication de cellulose est renvoyé dans le lessiveur (1).

8. Procédé selon une ou plusieurs des revendications 1 à 7, caractérisé en ce que le liquide de lessivage contenant des pentoses est envoyé dans l'installation de production de furfural (8, 10, 16) avec un temps de séjour global situé dans la plage de 10 à 60 min, de préférence de 10 à 25 min, le liquide de lessivage étant chauffé à une température comprise entre 130 et 180°C, de préférence entre 150 et 180°C, et étant maintenu dans cette plage.

9. Installation pour la mise en oeuvre du procédé selon une ou plusieurs des revendications 1 à 8, comportant un lessiveur (1) dans lequel débouche une conduite d'alimentation (2) pour la biomasse contenant de la lignocellulose et une conduite (2') pour la vapeur et auquel se raccordent une conduite d'évacuation (3) pour la biomasse traitée thermiquement dans le lessiveur (1) ainsi qu'une conduite ramifiée (5) qui aboutit à un échangeur de chaleur (4) duquel part une conduite de recyclage (6) pour le liquide de lessivage qui débouche dans le lessiveur (1), et une installation de production de furfural comprenant un réacteur tubulaire (8), une colonne de réaction (10) et un dispositif de distillation (16) situé en aval, dont le réacteur tubulaire (8) peut être relié au lessiveur (1) par une conduite d'alimentation (9) et dont la colonne de réaction (10) peut être reliée au lessiveur (1) par une conduite de recyclage (11).

10. Installation selon la revendication 9, caractérisée en ce que la conduite ramifiée (5) part d'un point H du lessiveur (1) au niveau duquel la concentration en pentoses est supérieure à 5 g/l et la décomposition des pentoses dans le liquide de lessivage est inférieure à 10 %.

11. Installation selon la revendication 9 ou 10, caractérisée en ce que le dispositif de distillation (16) peut être relié au lessiveur (1) par des conduites pour le recyclage du liquide séparé du furfural.

12. Installation selon une ou plusieurs des revendications 9 à 11, caractérisée en ce qu'une conduite d'évacuation de vapeurs (12) de la colonne de réaction (10) débouche dans un récipient collecteur (14) qui est relié au dispositif de distillation (16), le récipient collecteur (14) présentant un volume de gaz (18) qui peut être relié au lessiveur (1) par des conduites.

FIG. 1

FIG. 2

9